# EUROPÄISCHE PATENTANMELDUNG

(11) **EP 0 792 648 A2**
(43) Veröffentlichungstag der Anmeldung: **03.09.1997**
(21) Anmeldenummer: 97101251.3
(22) Anmeldetag: 28.01.1997
(51) Int. Cl.: A61K 38/58

(54) **Pharmazeutische Zusammensetzung enthaltend Hirudin und Verfahren zu deren Herstellung**

(30) Priorität: 27.02.1996 DE 19607239
(71) Anmelder: BEHRINGWERKE Aktiengesellschaft, 35001 Marburg (DE)
(72) Erfinder: Brazel, Dieter, Dr., 35041 Marburg (DE)
(74) Vertreter: Fischer, Hans-Jürgen, Dr.

(57) **Zusammenfassung**

Die vorliegende Erfindung betrifft eine pharmazeutische Zusammensetzung, die Hirudin und ein Salz einer Carbonsäure, jedoch im wesentlichen kein Magnesium- oder Kalziumsalz und auch kein Kaliumphosphat enthält.

## Beschreibung

Die vorliegende Erfindung betrifft eine pharmazeutische Zusammensetzung, die Hirudin und ein Salz einer Carbonsäure, jedoch im wesentlichen kein Magnesium- oder Kalziumsalz und auch kein Kaliumphosphat enthält.

Hirudin, ein aus Hirudo medicinalis isolierbares Polypeptid mit blutgerinnungshemmender Wirkung, ist eine Klasse von verwandten Polypeptiden mit der gleichen pharmazeutischen Wirkung. Vertreter dieser Klasse sind z. B. die Hirudinvariante 1 (HV1; EP-A-0 142 860), die Hirudinvariante 2 (HV2; EP-A-0 158 564), die Hirudinvariante 3 (PA; WO 86/03493), Thr-Tyr-Hirudin (Des-(Val)₂-Hirudin; EP-A-0 158 986), Leu-Thr-Hirudin (EP-A-0 324 712) und andere Hirudine wie beispielsweise in der EP-A-0 171 024 veröffentlicht. Der Begriff "Hirudin" bezieht sich somit nicht nur auf natürlich vorkommende Hirudinvarianten, wie z. B. HV1-Hirudin, sondern auch auf synthetische Hirudinvarianten, wie z. B. das Leu-Thr-Hirudin, Desulfatohirudinvarianten oder Mutanten davon, die die gerinnungshemmende Wirkung beibehalten haben. Beispiele hierzu sind in der WO 95/20399 offenbart.

Es ist bekannt, daß Hirudin in wässrigen Lösungen oder in pulvriger Form eine geringe Stabilität aufweist. So offenbart beispielsweise die WO 95/20399 eine gefriergetrocknete pharmazeutische Zusammensetzung, die Hirudin, ein Zucker und als Stabilisator Kaliumphoshat enthält. Die EP-A-0 665 019 offenbart hingegen eine gefriergetrocknete pharmazeutische Zusammensetzung, die Hirudin und ein wasserlösliches Salz von Kalzium und/oder Magnesium enthält. Diese beiden pharmazeutischen Zusammensetzungen haben jedoch den Nachteil, daß die Stabilisatoren zwar den Abbau von Hirudin verzögern, jedoch nicht im wesentlichen verhindern können. Auch wurde gefunden, daß die dort beschriebene RP-HPLC Methode zu unspezifisch ist, um Abbauprodukte abzutrennen und zu erfassen.

Aufgabe der vorliegenden Erfindung war daher, eine pharmazeutische Zusammensetzung zu finden, die den Abbau von Hirudin während eines überschaubaren Zeitraums im wesentlichen verhindert oder zumindest deutlich verzögert. Als ein überschaubarer Zeitraum werden in dem beispielhaft beschriebenen Testsystem bei 37 °C 2 Wochen bis 6 Monate, vorzugsweise 6 bis 12 Wochen angesehen.

Es wurde nun gefunden, daß eine pharmazeutische Zusammensetzung, die Hirudin und ein Salz einer Carbonsäure, jedoch im wesentlichen kein Magnesium- oder Kalziumsalz und auch kein Kaliumphoshat enthält, Hirudin deutlich besser stabilisieren kann als die oben beschriebenen pharmazeutischen Zusammensetzungen. Vorzugsweise ist das Salz der Carbonsäure ein Alkalisalz, insbesondere ein Natriumsalz. Auch ist es vorteilhaft, wenn die pharmazeutische Zusammensetzung zusätzlich einen Zucker, vorzugsweise Mannit, Trehalose, Sucrose, Sorbitol, Fructose, Glucose, Maltose, Lactose und/oder Dextran, vorzugsweise Mannit und/oder Trehalose, insbesondere Mannit als Gerüstbildner und zur Erreichung einer isotonischen Lösung enthält. Die Carbonsäure ist vorzugsweise Essigsäure, Oxalsäure, Äpfelsäure, Weinsäure, Glutaminsäure und/oder Zitronensäure, vorzugsweise Essigsäure, Äpfelsäure, Weinsäure und/oder Glutaminsäure, insbesondere Essigsäure und Glutaminsäure. In einer weiteren bevorzugten Ausführungsform der Erfindung handelt es sich bei der pharmazeutischen Zusammensetzung um eine gefriergetrocknete Zusammensetzung. Daher ist ein weiterer Gegenstand der Erfindung auch ein Verfahren zur Herstellung der erfindungsgemäßen pharmazeutischen Zusammensetzung, wobei die wässrigen Lösungen der einzelnen Bestandteile nach dem Vermischen gefriergetrocknet werden. Die Hirudinlösung hat im allgemeinen eine Konzentration von 0,1 bis 500 mg/ml, vorzugsweise 5 bis 100 mg/ml, insbesondere 10-60 mg/ml, vor allem ca. 10 mg/ml. Die Konzentration der Salzlösung der Carbonsäure ist im allgemeinen 10 bis 100 mM, vorzugsweise 20 bis 50 mM, insbesondere ca. 25 mM und gegebenenfalls hat die Zuckerlösung im allgemeinen eine Konzentration von 10 bis 100 mg/ml, vorzugsweise 20 bis 70 mg/ml, insbesondere ca. 40 mg/ml. Der pH-Wert beträgt nach dem Vermischen der Lösungen im allgemeinen 4 bis 9, vorzugsweise 6 bis 8, insbesondere ca. 7. Die vorliegende Erfindung umfaßt auch die allgemeine Verwendung eines Salzes einer Carbonsäure als Stabilisator zur Herstellung einer pharmazeutischen Zusammensetzung, insbesondere einer pharmazeutischen Zusammensetzung, die Hirudin und vorzugsweise einen Zucker enthält. Die oben genannten bevorzugten Ausführungsformen beziehen sich auch auf diese Verwendung.

Die folgenden Beispiele sollen die Erfindung näher erläutern:

### Beispiele:

### 1. RP-HPLC-Methode

Zur Analyse wird eine Nucleosil 120-3-C18 (250 x 4 mm) Säule von Machery & Nagel, FRG, verwandt. Mobile Phase A ist 5 Volumenprozent Acetonitrill Wasser. Mobile Phase B ist 70 Volumenprozent Acetonitril/Wasser. Beide Puffer enthalten 1 Volumenprozent Phosphorsäure (85 %), 0,2 Gewichtsprozent NaClO₄; pH 3 wird mit Triethylamin eingestellt. Die Detektion erfolgt bei 210 nm. Die Elution erfolgt bei 50 °C und einer Flußrate von 1 ml/min. Der lineare Gradient aus Laufmittel A und B beginnt bei 0 Minuten mit 23 % B und steigt in 45 Minuten auf 28 % B. Nach 10 Minuten bei 75 % B wird die Säule 20 Minuten lang bei 23 % B äquilibriert.

### 2. Beispiel I

Zu einer konzentrierten wässrigen Lösung von Hirudin ([Leu¹, Thr²]-63-desulfohirudin) wird a) Mannit; b) 2-Amino-2-(hydroxymethyl)-1,3-propandiol(Tris) und Mannit und c) Natrium-citrat und Mannit zugegeben (siehe Tabelle 1). Mannit dient in allen Fällen als Gerüstbildner und zur Erreichung einer isotonischen Lösung (ca. 290 mOsmol/kg). Die Konzentration von Hirudin ist jeweils 10 mg/ml. Der pH wird mit NaOH bzw. HCl auf pH 7 eingestellt.
Die Lösungen wurden gefriergetrocknet und bei 37 °C inkubiert Nach entsprechenden Inkubationszeiten wurden Proben entnommen, mit Wasser rekonstituiert (1 mg/ml) und der Hauptpeakgehalt per RP-HPLC ermittelt.

Folgende Resultate werden erhalten:

**Tabelle 1**

| | | | **Tage** | | | |
|---|---|---|---|---|---|---|
| | Puffersubstanz (25 mM) | Mannit (mg/ml) | 0 | 30 | 90 | 180 |
| | | | **% Hauptpeak-Fläche** | | | |
| | - | 50 | 96,0 | 94,6 | 94,0 | 92,8 |
| | Tris | 40 | 95,8 | 94,7 | 93,7 | 91,2 |
| | Natriumcitrat | 35 | 95,7 | 95,3 | 95,2 | 94,9 |

Der stabilisierende Effekt ist bei der carbonsäurehaltigen Substanz Natriumcitrat besser als bei Tris.

### 3. Beispiel II

Zu einer konzentrierten wässrigen Lösung von Hirudin wird 40 mg/ml Mannit als Gerüstbildner und zur Herstellung der Isotonizität zugegeben. Weiterhin werden folgende Substanzen mit Carbonsäurefunktionen zugegeben (Konzentration 25 mM): a) Natriumacetat; b) Natriumcitrat; c) Natrium-glutamat; d) Natriummalat; e) Natriumoxalat; f) Natriumtartrat. Der pH wird mit NaOH bzw. HCl auf pH 7 eingestellt. Die Konzentration von Hirudin ist jeweils 10 mg/ml.

Die Lösungen wurden gefriergetrocknet und bei 37 °C inkubiert. Nach entsprechenden Inkubationszeiten wurden Proben entnommen, mit Wasser rekonstituiert (1 mg/ml) und der Hauptpeakgehalt per RP-HPLC ermittelt.

**Tabelle 2**

| | **Tage** | | | |
|---|---|---|---|---|
| Natrium- | 0 | 14 | 42 | 84 |
| | **% Hauptpeakfläche** | | | |
| -acetat | 96,9 | 96,9 | 96,9 | 96,8 |
| -citrat | 97,1 | 96,6 | 96,6 | 96,5 |
| -glutamat | 96,8 | 96,7 | 96,7 | 96,9 |
| -malat | 96,9 | 96,7 | 96,7 | 96,8 |
| -oxalat | 97,0 | 96,3 | 95,8 | 95,4 |
| -tartrat | 96,7 | 96,5 | 96,5 | 96,6 |

Alle Carbonsäurehaltigen Substanzen zeigen einen stabilisierenden Effekt; wobei er sehr gut bei Citrat, Malat oder Tartrat und am stärksten bei Glutamat und Natriumacetat ausgeprägt ist.

## Patentansprüche

1. Pharmazeutische Zusammensetzung, enthaltend Hirudin und ein Salz einer Carbonsäure, jedoch im wesentlichen kein Magnesium- oder Kalziumsalz und auch kein Kaliumphosphat.

2. Pharmazeutische Zusammensetzung nach Anspruch 1, dadurch gekennzeichnet, daß die Carbonsäure Essigsäure, Oxalsäure, Äpfelsäure, Weinsäure, Glutaminsäure und/oder Zitronensäure, vorzugsweise Essigsäure, Äpfelsäure, Weinsäure und/oder Glutaminsäure, insbesondere Essigsäure und Glutaminsäure ist.

3. Pharmazeutische Zusammensetzung nach einem der Ansprüche 1 bis 2, dadurch gekennzeichnet, daß der Zucker Mannit, Trehalose, Sucrose, Sorbitol, Fructose, Glucose, Maltose, Lactose und/oder Dextran, vorzugsweise Mannit und/oder Trehalose, insbesondere Mannit ist..

4. Pharmazeutische Zusammensetzung nach einem der Ansprüche 1 bis 3, dadurch gekennzeichnet, daß das Salz der Carbonsäure ein Alkalisalz, vorzugsweise ein Natriumsalz ist.

5. Pharmazeutische Zusammensetzung nach einem der Ansprüche 1 bis 4, dadurch gekennzeichnet, daß die genannte Zusammensetzung gefriergetrocknet ist.

6. Verfahren zur Herstellung einer pharmazeutischen Zusammensetzung gemäß einem der Ansprüche 1 bis 5, dadurch gekennzeichnet, daß die wäßrigen Lösungen der einzelnen Bestandteile nach dem Vermischen gefriergetrocknet werden.

7. Verfahren nach Anspruch 6, dadurch gekennzeichnet, daß Hirudin in einer Konzentration von 0,1 bis 500 mg/ml, vorzugsweise 5 bis 100 mg/ml, insbesondere 10-60 mg/ml, vor allem ca. 10 mg/ml, das Salz der Carbonsäure 10 bis 100 mM, vorzugsweise 20 bis 50 mM, insbesondere ca. 25 mM und gegebenenfalls die Konzentration des Zuckers 10 bis 100 mg/ml, vorzugsweise 20 bis 70 mg/ml, insbesondere ca. 40 mg/ml ist.

8. Verwendung eines Stabilisators zur Herstellung einer pharmazeutischen Zusammensetzung, dadurch gekennzeichnet, daß der Stabilisator ein Salz einer Carbonsäure ist.

9. Verwendung nach Anspruch 8, dadurch gekennzeichnet, daß die pharmazeutische Zusammensetzung Hirudin und vorzugsweise einen Zucker enthält.
